Europäisches Patentamt

European Patent Office

Office européen des brevets

(11) Publication number: **0 231 634**
**A2**

## EUROPEAN PATENT APPLICATION

(21) Application number: 86309927.1

(51) Int. Cl.⁴: **A61M 25/00**

(22) Date of filing: **18.12.86**

(30) Priority: **18.12.85 JP 284729/85**

(43) Date of publication of application:
**12.08.87 Bulletin 87/33**

(84) Designated Contracting States:
**AT BE CH DE ES FR GB IT LI LU NL SE**

(71) Applicant: SHERWOOD MEDICAL COMPANY
1831 Olive Street
St. Louis, MO 63103(US)

(72) Inventor: Okada, Yosuke
2748-1, Ichinoniya Morimachi
Shuchi-Gun Shizuoka-Ken(JP)

(74) Representative: Chettle, Adrian John et al
c/o John Wyeth & Brother Limited
Huntercombe Lane South
Taplow Maidenhead Berkshire, SL6 0PH(GB)

(54) **Catheter obturator.**

(57) A catheter obturator (10) comprises a resilient tube (24) closed at one end and having the other end attached to a hub (12). A rod (26) is insertable through the hub (12) into the tube (24) to elongate the tube and thereby reduce the external diameter thereof. In the elongated condition the tube (24) may be easily inserted into a catheter; removal of the rod (26) causes the tube (24) to expand radially and block the catheter. A locking device (29, 34) to hold the rod and tube in the extended condition is disclosed. The tube may have a hardened tip (42).

EP 0 231 634 A2

FIG.1

Xerox Copy Centre

## Catheter Obturator

This invention relates to an improved catheter obturator.

Catheter obturators are inserted into catheters to prevent fluid flow therethrough; one known obturator comprises a solid rod of plastic material. In order for such an obturator to fully close the catheter bore it is desirable that the external diameter of the rod be equal to or slightly greater than the internal diameter of the catheter. However such obturators are extremely difficult to insert, and for practical reasons rod obturators having a diameter slightly smaller than the bore of the catheter are used. This has the disadvantage that body fluids may pass between the obturator and the catheter wall. Where the catheter is inserted in a blood vessel, blood may coagulate against the catheter wall.

It is an object of the invention to provide an improved catheter obturator which can easily be inserted into a catheter and yet fully close the bore thereof.

According to the invention there is provided a catheter obturator comprising a resilient tube closed at one end and attached at the other end to a hub having an opening therethrough into the tube, and a rod insertable through said opening into the tube, the rod being substantially longer than the tube.

The external diameter of the tube may depend on the diameter of the catheter into which it is to be inserted but the tube is always substantially greater in diameter than the respective catheter.

In use the rod is pushed through the hub to stretch the resilient tube; as the tube elongates its diameter reduces until it is less than the bore diameter of the catheter. The obturator can then be easily inserted into the catheter to the desired depth since there is no frictional resistance between the obturator and the catheter wall. Removal of the rod causes the resilient tube to expand radially against the catheter wall effectively sealing the bore thereof. The obturator may be removed by reversing the sequence of operations.

In the preferred embodiment locking means are provided between the rod and hub to hold the tube in the elongated condition. The locking means can be any suitable device such as a bayonet fitting or a screw thread.

The locking means may include a compressible annular bush to squeeze the rod and thereby assist retention thereof in the extended condition.

In a modified embodiment the tube has a hardened distal tip to prevent damage from the rod.

Other features of the invention will be apparent from the following description of a preferred embodiment shown by way of example only in the accompanying drawings in which:-

Figure 1 is a longitudinal section through a catheter obturator according to the present invention;

Figure 2 is a view similar to Figure 1, showing the obturator in an alternative condition;

Figure 3 is a longitudinal section through a catheter into which the obturator of Figures 1 and 2 is inserted;

Figure 4 is a fragmentary longitudinal section through the tip of a modified obturator; and

Figure 5 is a longitudinal section through a prior art obturator.

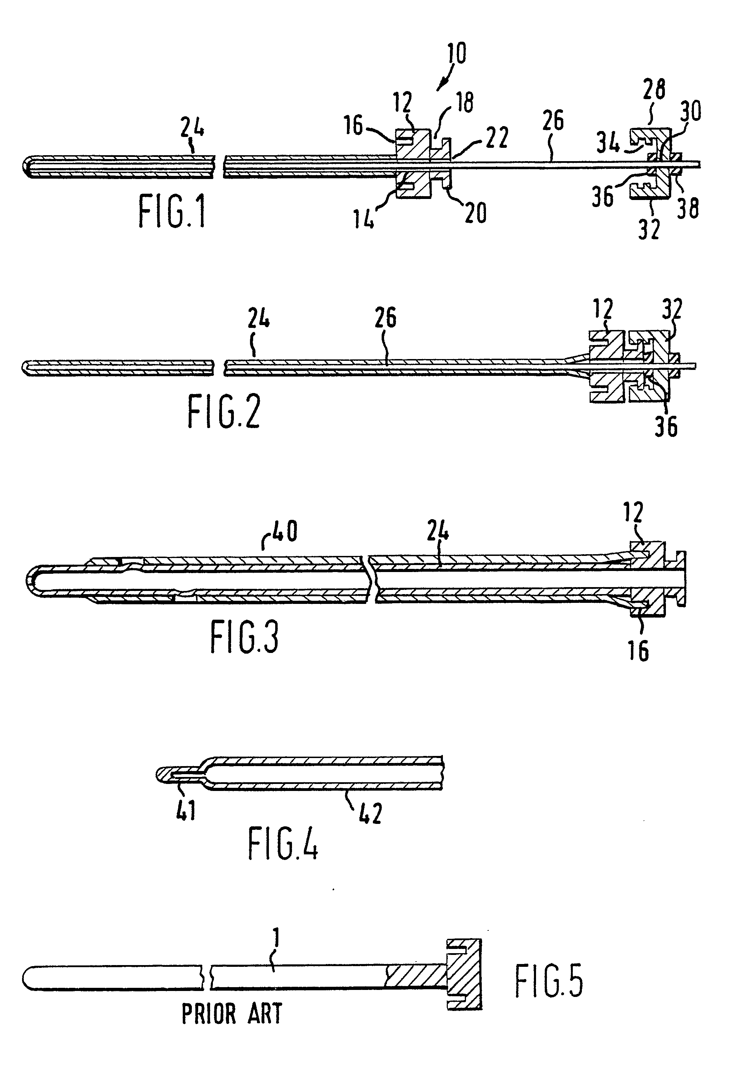

Referring to Figure 1, there is shown a catheter obturator 10 comprising a hub 12 having a bore 14 therethrough. The distal face of the hub 12 (the left side as shown in Figure 1) is provided with a circular groove 16 adapted to receive the proximal end of a catheter; the catheter may be inserted and retained in a blood vessel for example. The proximal face of the hub 12 includes an axially extending annular boss 18 having a radially extending flange formed at its proximal edge. The flange has locking means 20 at its radial periphery for co-operation with locking means of a cap to be described hereinafter; the locking means may be for example co-operating threads, a bayonet fitting or a snap fitting. The bore of the boss is co-axial with the bore of the hub.

The catheter obturator 10 also includes a tube 24 of a resilient material secured at one end to the distal face of the hub 12; the distal end of the resilient tube 24 is closed as shown. The external diameter of the tube 24 is sized to be somewhat larger than the bore diameter of the catheter into which it is to be inserted. A range of different tube diameters and lengths would be provided to suit different catheter bore sizes and lengths.

The catheter obturator 10 further comprises a rod assembly 28 which includes a rod 26 for insertion into the tube 24 through the bores 14 and 22 of the hub 12. The rod 26 is of smaller diameter than the bore diameter of the tube 24. The assembly 28 includes a cap 32 with an opening 30, through which the rod 26 passes. The cap 32 has a locking means 34 adapted to engage the locking means 20 on the flange. As shown, male locking means 20 are provided on the flange and the locking means 34 are female. Resilient annular bushes 36 and 38 are mounted on the opposite sides of the opening 30 and are sized to grip the rod 26. The rod 26 is relatively non-resilient compared to the tube 24 but is nevertheless flexible. The rod 26 and cap 32 may be separately provided.

Prior to use the rod 26 is passed through the cap 32 against the resistance of the bushes 36, 38. The rod 26 is then inserted into the tube 24 through the bores 14 and 22 in the hub 12. Further inward movement of the rod 26 stretches the tube 24; as the tube 24 elongates, the external diameter thereof reduces as shown in Figure 2. The tube 24 is held in the stretched condition by engagement of the co-operating locking means 20, 34. Engagement of the locking means compresses the bush 36 against the proximal face of the boss 18; the bush 36 is deformed to more firmly grip the rod 26. In the stretched condition the external diameter of the tube 24 is somewhat smaller than the bore diameter of the catheter into which it is to be inserted. The position of the cap 32 is chosen to suit the tube elongation required and may be preset or set according to, for example, graduations on the rod 26.

In use, the extended obturator 10 is inserted into a catheter; because the tube 24 is of smaller diameter than the catheter bore there is little resistance to insertion. When the tube 24 is in the desired position the locking means are released and the rod 26 is removed. The tube tends to return to its original shape under its own resiliency. As a result, the wall of the tube 24 engages the inner wall of the catheter 40 to fully close the bore thereof. This condition is illustrated in Figure 3, the tube bulges slightly into eyes of the catheter 40 as shown. The outer end of the catheter 40 may be engaged in the circular groove 16 on the distal face of the hub 12 to retain the tube 24 in place. The catheter is thus completely closed against leakage.

Figure 4 shows a modification of the preferred embodiment in which a tube 41 has a hardened tip 42 to prevent damage by the rod 16. The tip may be hardened by any convenient method or may be a separate attachment. The external diameter of the tip 42 is less than the bore diameter of the catheter for which the obturator is intended.

Figure 5 shows a prior art obturator comprising a solid rod of plastics material. The obturator has a proximal hub with a circular groove corresponding to groove 16.

Other variations are possible and it is intended that the invention be limited only by the scope of the claims appended hereto.

**Claims**

1. A catheter obturator (10) comprising a resilient tube (24) closed at one end and attached at the other end to a hub (12) having an opening (14) therethrough into said tube (24), and a rod (26) insertable through said opening (14) into the tube - (24), the rod (26) being substantially longer than said tube (24).

2. A catheter obturator according to Claim 1, wherein said opening (14) is co-axial with said tube (24).

3. A catheter obturator according to Claim 1 or Claim 2, wherein said rod (26) and hub (12) are provided with co-operating locking means (20, 34) engageable to hold said rod (26) against movement axially of said hub (14).

4. A catheter obturator according to Claim 3, wherein the locking means (20) of said hub (12) is male and the locking means (34) of said rod (26) is female.

5. A catheter obturator according to Claim 4, wherein the locking means (34) of said rod (26) comprises a cap (32) having an aperture (30) therethrough to receive said rod (26) and an annular elastic bush (36) co-axial with said aperture (30) and on the distal side thereof.

6. A catheter obturator according to Claim 5, wherein said bush (36) is sized to resiliently grip the rod (26).

7. A catheter obturator according to Claim 5 or Claim 6, wherein engagement of said locking means (20, 34) squeezes said bush (36) to reduce the cross-sectional area of the aperture therethrough.

8. A catheter obturator according to any preceding claim wherein a circular groove (16) is provided on the distal face of said hub (12) to receive one end of a catheter.

9. A catheter obturator according to any preceding claim, wherein said resilient tube (24) has a relatively hard distal end portion.

10. A catheter obturator according to Claim 9, wherein said end portion comprises a tubular tip - (42), the external diameter of said tip (42) being less than the external diameter of said tube (24).

FIG.1

FIG.2

FIG.3

FIG.4

FIG.5

PRIOR ART